# FASCICULE DE BREVET EUROPEEN

(11) **EP 3 082 700 B1**
(45) Date de publication et mention de la délivrance du brevet: **07.08.2019**
(21) Numéro de dépôt: 14830622.8
(22) Date de dépôt: 11.12.2014
(51) Int. Cl.: A61F 13/537, A61F 13/42, A61F 13/00

(54) **ARTICLE A APPLIQUER SUR LA PEAU, LES MUQUEUSES OU UNE PLAIE**
ARTIKEL ZUM AUFTRAGEN AUF DIE HAUT, AUF MUKOSEMEMBRANEN ODER AUF EINE WUNDE
ARTICLE TO BE APPLIED AGAINST THE SKIN, MUCOUS MEMBRANES OR A WOUND

(30) Priorité: 19.12.2013 FR 1363022
(43) Date de publication de la demande: 26.10.2016
(73) Titulaire: Commissariat à l'Énergie Atomique et aux Énergies Alternatives, 75015 Paris (FR); Urgo Recherche Innovation et Développement, 21300 Chenove (FR)
(72) Inventeur: REVOL-CAVALIER, Frédéric, F-38180 Seyssins (FR); MESSAOUD, Mouna, B.P. 1173 Sfax 3000 (TN); STEINBRUNN, Julien, F-21380 Messigny Et Vantoux (FR)
(74) Mandataire: Nony
(86) Numéro de dépôt international: PCT/IB2014/066789
(87) Numéro de publication internationale: WO 2015/092629

(56) Documents cités:
- EP-A1- 2 465 418
- WO-A2-2004/049992
- US-A- 4 507 121
- US-A1- 2010 305 490

## Description

La présente invention concerne les articles à appliquer sur la peau ou les muqueuses selon les revendications 1 à 15, et plus particulièrement mais non exclusivement, les pansements.

Il existe un besoin pour connaître le degré de remplissage d'un article absorbant, notamment d'un pansement appliqué sur une plaie faiblement exsudative, afin de pouvoir décider de son remplacement ou non, en vue de l'utiliser au mieux.

En effet, le fait de changer de pansement trop fréquemment peut conduire à un mauvais déroulement du processus de cicatrisation de la plaie, par perturbation de la cinétique de cicatrisation, mais aussi à un risque accru de contamination bactérienne.

Cependant, il est souvent conseillé de changer fréquemment de pansement, afin d'éviter tout contact prolongé avec un milieu trop humide pour la personne, quel que soit le patient considéré. Or, le niveau d'exsudation d'une plaie dépend beaucoup du patient et s'avère difficile à quantifier au préalable.

Dans le cas où le liquide à absorber est l'urine d'un jeune nourrisson et l'article une couche-culotte, il y a également un intérêt à maintenir l'article en place jusqu'à saturation, de façon à réduire la quantité d'articles utilisés ainsi que le travail nécessaire au changement de l'article par le personnel soignant ou par les parents.

L'invention vise à perfectionner encore les articles absorbants à appliquer sur la peau ou les muqueuses afin de pouvoir les utiliser dans des conditions les plus proches possible de l'optimum.

La demande internationale WO 2010/147533 décrit un pansement, qui n'est pas prévu pour signaler son degré de remplissage.

La demande de brevet européen EP 2 465 418 décrit un dispositif de détermination d'un débit d'excrétion d'un fluide corporel comportant un élément absorbant présentant au moins trois électrodes couplées électriquement deux à deux. L'élément absorbant peut comporter un révélateur colorimétrique de la présence de fluide corporel.

La demande de brevet américain US 2010/0305490 a pour objet une pansement permettant d'appliquer une pression réduite sur la zone à traiter comportant une couche interface en contact avec la peau, une première couche absorbante en communication fluidique avec la couche interface, une couche de déviation permettant de répartir la pression réduite sur la première couche absorbante, une deuxième couche absorbante en communication fluidique avec la couche de déviation et une pompe en communication fluidique avec la deuxième couche absorbante permettant de générer la pression réduite. Le pansement peut également comporter un indicateur visuel de la présence d'humidité dans la couche dans laquelle il est placé.

La demande de brevet américain US 4 507 121 divulgue une couche culotte comportant un ou deux indicateurs d'humidité formé par une zone contenant un indicateur chimique d'humidité. Le ou les indicateurs sont isolés de la peau de l'utilisateur par une structure capillaire permettant un flux de liquide d'une couche absorbante de la couche vers une poche isolée contenant un matériau absorbant par l'intermédiaire d'une ouverture capillaire entre les deux. Le flux de liquide est unidirectionnel dans la structure capillaire de sorte que l'indicateur chimique ne puisse pas diffuser dans l'ensemble de la couche.

La demande internationale WO 2004/049992 concerne une couche culotte comportant une couche absorbante non-amovible et une couche absorbante amovible en communication fluidique l'une avec l'autre. La couche absorbante amovible peut être disposé dans une poche ouvrable.

La demande internationale WO 2012/080865 concerne une couche culotte destinée au nourrisson, qui comprend une couche de ventilation permettant de réduire tout contact humide à l'interface avec la peau du nourrisson et de distribuer les liquides vers une couche absorbante adjacente; la structure comporte une couche de répartition qui permet d'homogénéiser le liquide sur l'ensemble de sa surface. La demande décrit un motif externe imprimé en deux dimensions, comprenant éventuellement un indicateur coloré, et permettant de visualiser la présence d'urine sécrétée.

Les indicateurs colorés décrits dans les demandes ci-dessus ne permettent pas de connaître précisément le degré de remplissage de l'article par le liquide émis, étant essentiellement binaire et permettant de visualiser s'il y a présence ou non d'urine.

La demande internationale WO 2013/114273 décrit un article absorbant avec une face de contact avec la peau, comprenant deux couches drainantes séparées par un film barrière hydrophobe présentant au moins une ouverture, une couche absorbante et un moyen de détection électrique des liquides, comprenant un réseau d'électrodes. La structure décrite dans ce document permet de visualiser grâce à un afficheur électronique le taux de remplissage d'un pansement par exemple. Le film barrière hydrophobe comprend de préférence une seule perforation, située dans le même axe que la plaie sous-jacente.

Le dispositif de détection décrit dans ce document est trop coûteux et complexe pour pouvoir être intégré dans des articles à usage unique et à large diffusion comme des pansements, des couches culottes ou encore des produits d'hygiène intime.

La présente invention vise à fournir un article absorbant, dont le remplissage se fasse préférentiellement de façon relativement homogène, et présentant au moins un moyen de visualisation de l'évolution de ce remplissage.

L'invention a ainsi pour objet, selon l'un de ses aspects, un article à appliquer sur la peau, les muqueuses ou une plaie, pour y absorber un liquide, notamment un exsudat, comportant:
- une couche absorbante distale,
- une pluralité d'indicateurs visuels discrets changeant d'aspect en cas de contact avec le liquide, ces indicateurs visuels étant en contact avec la couche absorbante distale,
- une interface destinée à contacter la peau ou les muqueuses,
- entre l'interface et la couche absorbante distale, au moins une couche absorbante proximale, adjacente à l'interface,
- entre la couche absorbante proximale et la couche absorbante distale, au moins une couche formant barrière à l'eau, pourvue d'au moins un passage permettant au liquide ayant traversé la couche absorbante proximale de gagner la couche absorbante distale,
la capacité d'absorption d'eau de la couche absorbante proximale (10) étant supérieure à celle de la couche absorbante distale (20).

Le fait que l'article comporte une pluralité d'indicateurs visuels discrets permet de repérer la présence de liquide à différents endroits de la couche absorbante distale, et ainsi d'affiner la quantification du liquide absorbé par l'article. On dispose ainsi d'un article permettant une visualisation progressive de son taux de remplissage, qui donne une information représentative du niveau de saturation de l'article à tout moment. Ces indicateurs sont répartis dans la couche absorbante distale, ou à la surface externe de cette dernière, de telle sorte qu'ils sont visibles lorsque l'article est appliqué contre la peau.

Outre l'intérêt présenté pour un pansement, un article selon l'invention permet de définir le volume de sécrétion urinaire dispensé par le jeune nourrisson, grâce à la visualisation du niveau de saturation de l'article absorbant. Cela peut faciliter l'analyse du niveau de déshydratation du nourrisson.

L'épaisseur de la couche absorbante proximale est par exemple comprise entre 1 et 5 mm.

La capacité d'absorption d'eau de la couche absorbante proximale est de préférence supérieure d'un facteur 2 à celle de la couche absorbante distale, mieux au moins 5 fois supérieure. Cela permet d'éviter de créer un effet de pompage et de conserver l'essentiel du liquide dans la couche absorbante proximale. De la sorte, la couche absorbante proximale est remplie avant que la couche absorbante distale ne le soit complètement.

La couche absorbante distale a vocation à détecter le niveau de remplissage de l'article, non à stocker la majorité du liquide absorbé. En particulier, il est souhaitable de ne pas saturer trop vite la couche absorbante distale et de ne pas faire réagir tous les indicateurs visuels tant que la couche absorbante proximale n'est pas complètement remplie.

### Couche formant barrière

De préférence, la couche formant barrière est constituée par un film d'un matériau imperméable, notamment un film d'une matière thermoplastique, en particulier de polyuréthane, par exemple un film tel que celui commercialisé par la société PLASTO sous la désignation P1166.

De préférence, la couche formant barrière à l'eau est perméable à la vapeur d'eau. Ainsi, la couche formant barrière présente de préférence une perméabilité à la vapeur d'eau supérieure ou égale à 500 g/m²/24h, mesurée selon la norme NF EN 13726-2. D'une façon générale, la perméabilité à un gaz, voire à la vapeur d'eau, permet une meilleure aération de la plaie.

Le passage précité est de préférence excentré. Cela permet d'obtenir une meilleure répartition du liquide au sein de l'article, en obligeant le liquide à se répartir au sein de la couche absorbante proximale avant de passer dans la couche absorbante distale. En particulier, cela tend à empêcher que le liquide absorbé soit réparti autour d'un axe unique, comme dans le cas où il y aurait un passage central situé en face de la source du liquide, et cela permet une indication plus fine du niveau de remplissage de l'article par le ou les indicateurs visuels au contact de la couche absorbante distale.

Le passage est ainsi, de préférence, unique, ce qui permet de mieux prévoir et contrôler la répartition du liquide.

De préférence, le ou les passages présentent chacun une plus grande dimension transversale comprise entre 100 µm et 1cm, mieux entre 100 µm et 5 mm. Ce ou ces passages peuvent être de section circulaire.

L'épaisseur de la couche formant barrière peut être comprise entre 20 µm et 100 µm.

La couche formant barrière peut être unique ; en variante, l'article comporte plusieurs couches imperméables, par exemple laminées ensemble.

### Couches absorbantes

Les couches absorbantes distale et proximale présentent chacune de préférence une capacité d'absorption d'eau supérieure ou égale à 500g/m², mieux à 800g/m². Elles peuvent comporter ou être constituées de tout matériau apte à stocker les liquides, comme par exemple les matériaux utilisés dans le domaine de l'hygiène et des pansements.

La couche absorbante proximale ou distale comporte de préférence une mousse poreuse hydrophile, de préférence une mousse de polyuréthane, à l'image par exemple de celle commercialisée par les laboratoires URGO sous la dénomination Cellosorb ® ou encore de celle connue sous la dénomination MCOF.03 et commercialisée par la société Advanced Médical Solution (AMS).

La couche absorbante proximale est une couche de stockage du liquide absorbé.

Les couches absorbantes proximale et distale peuvent encore comporter des matériaux à base de polymère super absorbant (SAP), comme par exemple les non tissés absorbants incorporant des particules de SAP couramment utilisés dans le domaine de l'hygiène, les textiles absorbants comme par exemple les non tissés à base de viscose, de rayonne ou de cellulose, tels que par exemple une ouate, ou des hydrogels.

On préfère l'utilisation de non tissés obtenus par la méthode de fabrication par voie sèche, connue sous le nom de voie aérodynamique ou "airlaid", qui contiennent des particules de SAP et en particulier entre 20 et 60% en poids de SAP par rapport au poids total de non tissé.

La couche absorbante proximale peut ainsi être formée par un matériau super absorbant non tissé, par exemple tel que celui commercialisé par la société EAM Corporation sous la référence Novathin ®. Cela lui confère une capacité d'absorption élevée.

Selon une variante, on emploie comme matériau à base de SAP un matériau constitué de deux voiles cellulosiques entre lesquels sont incorporées des particules de polymères super absorbants, seules ou en association avec des liants.

Selon une autre variante, on utilise un matériau à base de fibres de SAP seules ou en association avec des fibres non absorbantes. De préférence, ce matériau se présente sous forme d'un non tissé.

### Indicateurs visuels

Les indicateurs visuels sont de préférence disposés avec un écartement constant dans au moins une direction, par exemple sous la forme d'un quadrillage, notamment avec un écartement compris entre 5 mm et 2 cm entre les centres de deux indicateurs.

Les indicateurs visuels sont par exemple constitués de pastilles déposées sur la couche absorbante distale, mais peuvent aussi être déposés par impression, par enduction, ou sous la forme d'une couche présentant des disparités de concentration d'un composé produisant une couleur au contact d'eau.

Les indicateurs visuels peuvent être séparés les uns des autres par de l'air ou par un matériau hydrophobe qui peut contribuer à maintenir les indicateurs en place, le cas échéant, notamment lorsqu'il s'agit de pastilles. Chaque pastille peut être au contact d'une matrice de confinement poreuse et perméable à l'eau.

Les indicateurs visuels sont par exemple des indicateurs colorés aptes à changer de couleur suivant le degré d'hydratation de la portion de couche absorbante distale à leur contact.

Il peut encore s'agir de pastilles de deux composés incolores dont l'un au moins est soluble dans le liquide absorbé, qui réagissent entre eux pour former un produit coloré lorsque l'un au moins passe en solution en présence de liquide. En variante, le ou les composés utilisés pour réaliser chaque indicateur visuel sont initialement colorés, et c'est le produit formé après mise en contact avec le liquide qui est incolore ou change de couleur.

Il peut également s'agir d'un ou plusieurs composés acido-basiques de pKa sensiblement neutre dont la forme acide n'a pas la même couleur que la forme basique. Ce ou ces composés peuvent être couplés à un acide faible et/ou une base faible qui passe en solution en présence de liquide. Il peut encore s'agir d'un ou plusieurs composés légèrement oxydants et/ou réducteurs.

La couleur des indicateurs visuels est de préférence générée par absorption dans le spectre visible, de façon à permettre l'évaluation du taux de remplissage de l'article sans avoir recours à un appareillage supplémentaire, tel qu'une lampe à rayonnement ultraviolet.

La couleur produite par un indicateur visuel peut correspondre à différentes teintes en fonction de la quantité de liquide absorbée par l'article, ce qui permet un suivi progressif du taux de remplissage de l'article. Par exemple, la couleur peut passer du blanc au rouge clair, puis du rouge clair au vermillon, du vermillon au rouge sang, et du rouge sang au bordeaux, en fonction du degré de saturation en eau. Un exemple d'indicateur visuel, prenant différentes teintes selon la quantité de liquide absorbée, est connu sous la référence HB Fuller full care 9390.

Le nombre d'indicateurs visuels va par exemple de 1 à 100.

L'article peut comporter des graduations, par exemple imprimées, qui sont associées à certains indicateurs visuels au moins et qui renseignent sur le taux de remplissage correspondant.

### Autres couches

L'interface avec la peau ou les muqueuses peut être définie par une couche de contact, comportant par exemple une trame textile. La couche de contact est de préférence microadhérente. Par exemple, il s'agit d'une trame textile enduite d'un gel, comme décrit dans les documents WO201240378 ou WO201240377.

Par couche de contact microadhérente, on entend qu'elle permet de se fixer provisoirement sur la plaie, cette couche pouvant être retirée sans que la structure de la plaie ou de la peau péri-lésionnelle ne soit altérée, si bien que ladite couche est repositionnable et facilite les soins infirmiers.

L'article peut comporter, entre la couche de contact et la couche absorbante proximale, au moins une première couche de répartition.

En effet, il est souhaitable que le liquide soit aussi bien réparti que possible, afin de pouvoir stocker davantage de liquide dans la couche absorbante proximale tout en évitant un reflux de liquide vers la peau, la plaie ou les muqueuses au contact de l'article.

En outre, il est souhaitable que le liquide soit réparti de façon homogène afin de pouvoir évaluer de façon fiable la quantité de liquide absorbée, grâce à la quantité de liquide passant dans la couche absorbante distale.

L'article peut aussi comporter, entre la couche absorbante proximale et la couche imperméable, une deuxième couche de répartition. Cela permet d'améliorer encore la répartition du liquide dans la couche absorbante proximale.

La capacité d'absorption des couches de répartition est relativement faible par rapport à celle de la couche absorbante proximale, de préférence entre 5 et 10 fois inférieure.

Dans le cas où l'article comporte une deuxième couche de répartition au contact de la couche formant barrière et de la couche absorbante proximale, il est souhaitable que le liquide ne puisse pas passer directement de la deuxième couche de répartition à la couche absorbante distale, mais circule de la deuxième couche de répartition à la couche absorbante proximale, puis de la couche absorbante proximale à la couche absorbante distale. A cet effet, la deuxième couche de répartition peut être rendue localement hydrophobe, notamment par enduction d'une matière occlusive, par exemple thermoplastique, par exemple du PVC éventuellement dissout dans un solvant approprié, tel que l'acétonitrile, au niveau d'une zone située en périphérie du ou des passages. En variante, la deuxième couche de répartition présente un ajour en vis-à-vis de chacun des passages. La section de chacun de ces ajours est de préférence supérieure à celle du passage correspondant.

La ou les couches de répartition peuvent être constituées de matériaux permettant l'étalement des liquides par diffusion transversale ou longitudinale et/ou les deux. De telles couches sont par exemple en des matériaux textiles ou non-tissé hydrophiles à base de fibres de polyester ou de polyoléfines. A titre d'exemple, on peut citer les produits commercialisés respectivement par les sociétés ORSA et FREUDENBERG sous les dénominations Jettex ® 1205c et Evolon ®.

L'article peut encore comporter une couche extérieure de protection, de préférence laissant les indicateurs visuels observables et ainsi non opaque, se superposant à la couche absorbante distale. Cette couche extérieure de protection est de préférence imperméable à l'eau, et présente une perméabilité à la vapeur d'eau supérieure ou égale à 500 g/m²/24h. De telles couches extérieures de protection sont couramment utilisées dans la réalisation de pansements et sont par exemple constituées de films de polyuréthane tels que les films commercialisés par la société Exopack Advanced Coating sous la désignation INSPIRE. Cette couche de protection est de préférence translucide.

La couche de protection peut être assemblée au reste de l'article au moyen d'un adhésif, de préférence discontinu, de manière à ne pas affecter la perméabilité de la couche à la vapeur d'eau.

L'article est de préférence un pansement, notamment conditionné à l'état stérile.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 est une vue en coupe, schématique et partielle, d'un article réalisé conformément à l'invention,
- la figure 2 est une vue analogue à la figure 1 d'une variante de réalisation,
- la figure 3 est une vue en coupe selon III-III de l'article de la figure 1,
- la figure 4 représente l'évolution de la quantité de marqueur colorimétrique ayant changé de couleur en fonction du taux de saturation du réservoir d'un article selon l'invention,
- les figures 5A et 5B sont des vues analogues aux figures 1 et 2 d'autres variantes d'articles selon l'invention,
- la figure 6 est une coupe selon VI-VI de l'article de la figure 5,
- la figure 7 représente une courbe analogue à celle de la figure 4 dans le cas d'un autre article selon l'invention,
- les figures 8 à 10 sont des vues analogues à la figure 1 de variantes utilisées pour réaliser des essais comparatifs, et
- la figure 11 est une coupe selon XI-XI de la figure 10.

Sur les figures, les proportions réelles des différents éléments constitutifs n'ont pas toujours été respectées, dans un souci de clarté du dessin. Certains éléments n'ont pas été représentés en contact, alors qu'ils le sont en réalité. De plus, certains constituants, par exemple des adhésifs, ont pu ne pas être représentés.

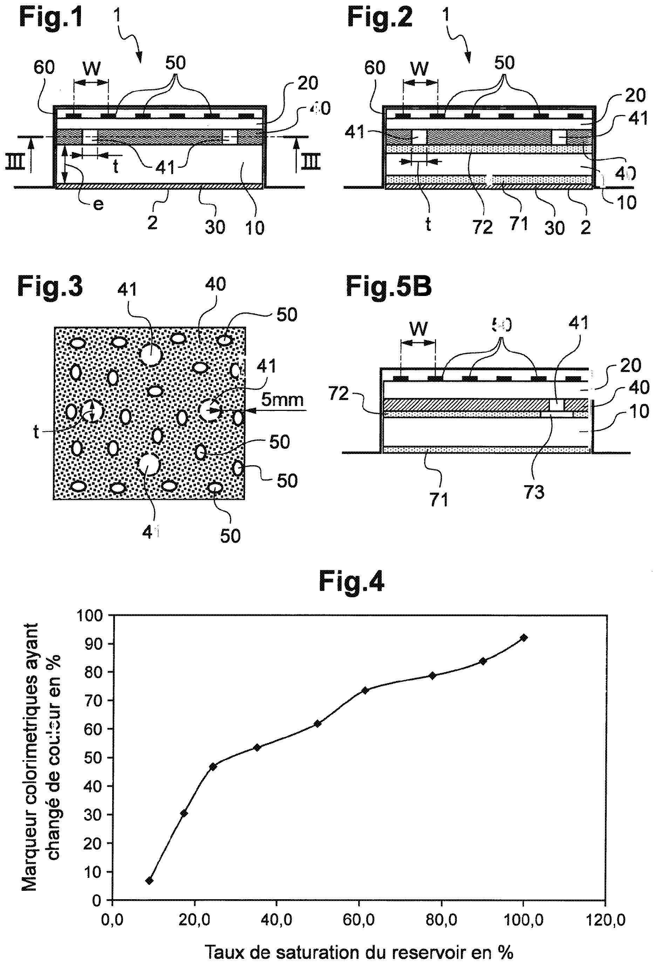

L'article absorbant 1 selon l'invention, représenté à la figure 1, est à appliquer par une face proximale 2 sur la peau saine ou lésée, et/ou sur les muqueuses. Il s'agit de préférence d'un pansement, mais la description qui suit vaut également pour un article autre, tel qu'une couche culotte ou un article d'hygiène intime.

L'article 1 comporte une ou plusieurs couches absorbantes capables d'accumuler le liquide reçu par la face proximale 2, par exemple l'exsudat d'une plaie.

L'article 1 comporte ainsi, comme illustré, une première couche absorbante 10, dite couche absorbante proximale ou réservoir, qui s'étend parallèlement à la face proximale 2, dans les deux dimensions, à savoir la longueur de l'article et sa largeur lorsque l'article présente une forme allongée.

La couche absorbante proximale 10 s'étend sur une épaisseur e qui est de préférence constante et supérieure ou égale à 1 mm.

Le contact avec la peau ou les muqueuses peut être défini par une couche d'interface 30 dont la face proximale 2 s'étend sous la couche absorbante proximale 10.

L'article 1 comporte également une deuxième couche absorbante 20, dite couche absorbante distale, qui s'étend de préférence sur la même étendue que la couche absorbante proximale 10.

Une couche intermédiaire formant barrière 40, imperméable à l'eau, s'étend entre les couches absorbantes proximale 10 et distale 20. Cette couche intermédiaire 40 est pourvue d'au moins un passage 41 permettant au liquide contenu dans la couche proximale 10 de gagner la couche distale 20. Ce passage est réalisé par exemple sous la forme d'un trou à travers le matériau de la couche intermédiaire 40.

La couche intermédiaire 40 peut comporter plusieurs passages 41, disposés par exemple comme illustré à la figure 3, chacun à mi-longueur d'un côté, et plus proche de ce côté que du centre de l'article.

Le ou les passages 41 peuvent tous avoir la même plus grande dimension transversale t. En particulier, le ou les passages 41 peuvent avoir des sections circulaires de diamètre t compris entre 100 µm et 5 mm.

Les passages 41 peuvent respecter dans leur disposition une symétrie axiale, mais d'autres agencements sont possibles.

L'article 1 comporte une pluralité d'indicateurs visuels 50, visibles lorsque l'article 1 est en place sur le corps, afin de permettre à l'observateur d'évaluer le degré de remplissage de l'article 1 et le besoin éventuel de le remplacer.

Ces indicateurs 50 sont de préférence, comme illustré, répartis de façon discrète à la surface de la couche absorbante distale 20, par exemple selon une disposition matricielle telle qu'illustrée à la figure 3.

La répartition des indicateurs 50 peut se faire en fonction de la disposition du ou des passages 41 traversant la couche imperméable 40, de façon à ce que le nombre d'indicateurs visuels 50 ayant changé d'aspect suite à la présence de liquide soit le plus fidèlement possible représentatif du degré de remplissage de l'article.

La répartition des indicateurs 50 peut également se faire selon un quadrillage avec un écartement *W* entre les indicateurs constant dans au moins une direction. L'écartement *W* est par exemple compris entre 5 mm et 2 cm, notamment de l'ordre de 1 cm.

L'article 1 est avantageusement recouvert d'une couche de protection extérieure 60, protégeant la couche absorbante distale 20 du milieu extérieur.

La couche de protection 60 est par exemple réalisée en une matière thermoplastique, notamment du polyuréthane, et présente de préférence une perméabilité à la vapeur d'eau supérieure à 500g : m²/24h, de façon à ne pas empêcher l'évaporation du liquide stocké et la respiration de la peau.

La couche de protection 60 est assemblée au reste de l'article par tout moyen approprié, de préférence au moyen d'un adhésif discontinu, non représenté.

Lors de l'utilisation de l'article 1, le liquide qui remplit la couche absorbante proximale 10 gagne par les passages 41 la couche absorbante distale 20. Les indicateurs visuels 50 changent d'aspect au fur et à mesure que le liquide s'étend dans la couche absorbante distale 20.

Afin d'homogénéiser la répartition du liquide, l'article 1 peut comporter des couches de répartition 71 et 72 respectivement situées en dessous et au-dessus de la couche absorbante proximale 10, comme illustré à la figure 2.

Le rôle des couches de répartition 71 et 72 n'est pas de stocker les liquides mais de favoriser leur étalement.

Les couches de répartition 71 et 72 sont de préférence moins absorbantes d'une part que la couche absorbante proximale 10 et d'autre part que la couche absorbante distale 20.

Les couches de répartition 71 et 72 présentent ainsi, de préférence, une capacité d'absorption d'eau qui est inférieure à 500 g/m².

De préférence, l'épaisseur de chacune de ces couches 71 et 72 est comprise entre 50 et 500 µm, de préférence entre 250µm et 500 µm.

De préférence encore, chaque couche de répartition 71 ou 72 comporte ou est constituée d'une couche d'un matériau hydrophile.

On peut utiliser, pour réaliser les couches de répartition 71 et 72 des matériaux à base de fibres absorbantes d'origine végétale, telle que la viscose, la cellulose ou ses dérivés. Ces matériaux peuvent se présenter sous forme de tricots, de tissés ou de non tissés, obtenus par voie sèche ou par voie humide, comme les papiers.

Afin d'évaluer les performances d'un article 1 selon l'invention, on a mesuré le taux de saturation de la couche absorbante proximale 10 en fonction du nombre d'indicateurs 50 ayant été activés, pour un article ayant la structure représentée à la figure 2.

Les résultats sont reportés à la figure 4. Dans cet exemple, l'interface 30 est constituée d'un non-tissé de type Bérotex ®, les couches absorbantes 10 et 20 sont constituées de mousse de polyuréthane et présentent des épaisseurs respectives de 4 et 2 mm, la couche imperméable 40 est constituée d'un film de polyuréthane, et l'article présente deux couches de répartition 71 et 72 dans un non tissé hydrophile de type Berkshire DR870 ®. Les indicateurs 50 utilisés sont des indicateurs colorés de type HB Fuller full care 9390 ®. La couche externe de protection 60 est un film polyuréthane, éventuellement enduit d'une masse adhésive telle qu'un adhésif acrylique, ou qu'une silicone.

Le débit d'arrivée de liquide a été fixé à 100µl/min sur un fritté de 3cm de diamètre, de façon à simuler une plaie de taille moyenne. Le pansement est de forme carrée, de 81 cm² d'étendue. Une surpression de 15 mbar par rapport à la pression atmosphérique est appliquée sur le pansement.

La couche absorbante proximale a été pesée régulièrement de façon à établir un lien entre le nombre d'indicateurs ayant été activés et le niveau de saturation de cette couche.

Des photographies des indicateurs sont effectuées régulièrement afin d'évaluer le nombre d'indicateurs ayant été activés. On voit sur la figure 4 que la progression du nombre d'indicateurs ayant changé d'aspect est un indicateur du taux de saturation.

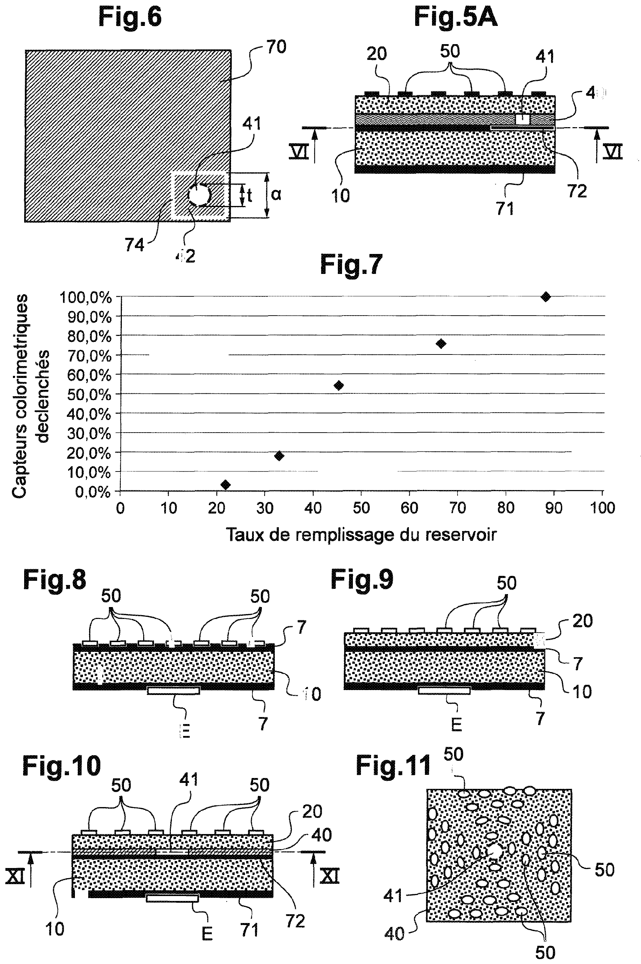

On s'intéresse maintenant aux exemples de réalisation représentés aux figures 5A, 5B et 6.

Afin de s'assurer que le liquide émis est transféré dans la couche absorbante distale 20 uniquement par la couche absorbante proximale 10, et non par la deuxième couche de transfert 72 située au-dessus de la couche absorbante proximale 10, la couche de répartition 72 peut être enduite d'un matériau barrière 42 depuis la deuxième couche de répartition 72 au niveau du passage 41, comme illustré à la figure 5A. Il s'agit par exemple de PVC dissout dans l'acétonitrile, d'une silicone PDMS Sylgar 184 de la société Dow Corning ®, ou une résine polymérisable de type Hoechst AZ4620, Hoechst AZ4562, Shipley 1400-17, Shipley 1400-27 ou Shipley 1400-37. Le matériau barrière 42 bloque le transfert du liquide dans la couche absorbante distale 20.

Cela permet de retarder le déclenchement des premiers indicateurs colorés et d'affiner la mesure du remplissage pour des taux de saturation plus élevés de la couche absorbante proximale.

Dans la variante illustrée à la figure 5B, la deuxième couche de répartition 72 comporte un ajour 73 en vis-à-vis du passage 41 et de sa périphérie, de façon à empêcher la propagation directe du liquide depuis la couche de répartition 72 vers la couche absorbante distale 20 par le passage 41.

On voit sur la figure 6 que le passage 41 peut être unique et situé à proximité d'un coin 74 de l'article, étant plus proche de ce coin que du centre de l'article. La figure 7 retrace une évolution similaire à la figure 4, dans le cas où le passage 41 est excentré, comme à la figure 6. On remarque que pour les faibles taux de remplissage, notamment inférieurs à 30%, le nombre d'indicateurs ayant changé d'aspect est moins important lorsque le passage est excentré. Le fait d'excentrer le passage du liquide retarde l'activation des premiers indicateurs.

Le matériau barrière 42 s'étend par exemple dans le sens de la largeur et de la longueur de l'article sur une distance *d* au moins double de la plus grande dimension *t* du passage 41.

### Essais comparatifs

Les configurations illustrées aux figures 8 à 11 représentent des structures visant à mettre en évidence certains avantages apportés par des particularités de réalisation.

La structure représentée à la figure 8 correspond à un article non conforme à l'invention ne comprenant qu'une seule couche absorbante 10, disposée entre et au contact de deux couches de répartition inférieure 71 et supérieure 72. La couche de répartition inférieure 71 est au contact d'une source émettrice de liquide E, et la couche de répartition supérieure 72 porte une pluralité d'indicateurs visuels 50. Cette configuration qui se caractérise notamment par l'absence de la couche imperméable induit une activation trop précoce des indicateurs 50.

La structure illustrée à la figure 9 diffère de celle de la figure 8 par la présence d'une couche absorbante distale 20, ajoutée entre la couche de répartition supérieure 72 et les indicateurs visuels 50. Cette configuration permet un meilleur suivi du remplissage de la couche absorbante proximale 10. Cependant, le liquide commence à se répartir entre les deux couches absorbantes 10 et 20 dès que le liquide atteint la couche de répartition supérieure 72. Cette répartition accélère le remplissage de la couche absorbante distale 20, ce qui a pour conséquence d'activer les indicateurs visuels 50 trop rapidement, d'une manière non représentative du taux de remplissage de la couche absorbante proximale 10. Ceci met en évidence l'intérêt de la couche formant barrière 40 d'une structure selon l'invention.

A la figure 10, qui correspond à une structure selon l'invention, une couche formant barrière 40 d'un matériau hydrophobe présentant un passage 41 en son centre est ajoutée à la structure de la figure 9, entre la couche de répartition supérieure 72 et la couche absorbante distale 20. Cette structure permet de mieux suivre le remplissage de la couche absorbante proximale 10. Cependant, la position centrale du passage 41 permet au liquide de passer dans la couche absorbante distale avant que la couche absorbante proximale soit complètement mouillée : cette configuration tend à induire une répartition axiale du liquide absorbé et une activation précoce des indicateurs 50. On a fait apparaître à la figure 11 la répartition des indicateurs visuels 50 dans cette configuration. L'essai correspondant à la figure 10 fait apparaître l'intérêt d'un positionnement excentré du ou des passages 41 lorsque la source de fluide est centrale.

L'invention n'est pas limitée aux exemples qui ont été décrits. En particulier, les différentes couches peuvent comporter d'autres matériaux que ceux évoqués, notamment choisis parmi ceux utilisés usuellement dans la fabrication des pansements, des couches-culottes, des articles d'hygiène intime, et des articles absorbants en général. La disposition et l'organisation des différents passages 41 peut être adaptée en fonction de la géométrie de l'article et du positionnement attendu de la source de liquide afin d'optimiser au mieux ses performances. De même, le choix des indicateurs visuels et leur répartition peuvent être modifiés sans sortir du champ de l'invention.

Le terme « comporte un » est synonyme de « comporte au moins un », sauf si le contraire est précisé.

## Revendications

1. Article (1) à appliquer sur la peau, les muqueuses ou une plaie, pour y absorber un liquide, notamment un exsudat, comportant:
- une couche absorbante distale (20),
- une pluralité d'indicateurs visuels (50) discrets changeant d'aspect en cas de contact avec le liquide, ces indicateurs visuels (50) étant en contact avec la couche absorbante distale (20),
- une interface (30) destinée à contacter la peau ou les muqueuses,
- entre l'interface (30) et la couche absorbante distale (20), au moins une couche absorbante proximale (10) adjacente à l'interface (30),
- entre la couche absorbante proximale (10) et la couche absorbante distale (20), au moins une couche formant barrière à l'eau (40) pourvue d'au moins un passage (41), de préférence unique et/ou excentré, permettant au liquide ayant traversé la couche absorbante proximale (10) de gagner la couche absorbante distale (20),
la capacité d'absorption d'eau de la couche absorbante proximale (10) étant supérieure à celle de la couche absorbante distale (20).

2. Article selon la revendication 1, le ou les passages (41) présentant chacun une plus grande dimension transversale (t) comprise entre 100 µm et 1cm, mieux entre 100 nm et 5 mm.

3. Article selon la revendication précédente, les indicateurs visuels (50) étant disposés avec un écartement constant dans au moins une direction, notamment avec un écartement (M/) compris entre 5 mm et 2 cm.

4. Article selon l'une quelconque des revendications précédentes, les indicateurs visuels (50) étant constitués de pastilles déposées sur la couche absorbante distale.

5. Article selon l'une quelconque des revendications précédentes, les indicateurs visuels (50) étant séparés les uns des autres par un matériau hydrophobe.

6. Article selon l'une quelconque des revendications précédentes, la couche absorbante distale (20) comportant une mousse poreuse hydrophile, de préférence une mousse de polyuréthane.

7. Article selon l'une quelconque des revendications précédentes, la couche formant barrière (40) étant constituée par un film d'un matériau imperméable, notamment un film d'une matière thermoplastique, en particulier de polyuréthane.

8. Article selon l'une quelconque des revendications précédentes, la couche proximale absorbante (10) comportant une mousse d'un matériau poreux hydrophile, notamment du polyuréthane.

9. Article selon l'une quelconque des revendications précédentes, la capacité d'absorption d'eau de la couche absorbante proximale (10) étant 2 fois supérieure, mieux au moins 5 fois supérieure à celle de la couche absorbante distale (20).

10. Article selon l'une quelconque des revendications précédentes, l'interface (30) étant définie par une couche de contact, de préférence constituée d'un non tissé, de préférence l'article comportant entre la couche de contact et la couche absorbante proximale (10) au moins une première couche de répartition (71).

11. Article selon la revendication précédente, comportant entre la couche absorbante proximale (10) et la couche formant barrière (40) une deuxième couche de répartition (72), la deuxième couche de répartition (72) étant de préférence rendue localement hydrophobe, notamment par une enduction de PVC, au niveau d'une zone (74) située en vis-à-vis de la périphérie du ou des passages (41) et optionnellement présentant un ajour (73) en vis-à-vis du ou des passages (41).

12. Article selon l'une quelconque des revendications 10 et 11, la ou les couches de répartition (71,72) comportant un textile ou un non tissé hydrophile, notamment à base de fibres polyester ou de fibres en polyoléfine.

13. Article selon l'une quelconque des revendications précédentes, la couche formant barrière (40) présentant une perméabilité à la vapeur d'eau supérieure ou égale à 500 g/m²/24h.

14. Article selon l'une quelconque des revendications précédentes, comportant une couche de protection (60), se superposant à la couche absorbante distale (20), imperméable à l'eau et présentant une perméabilité à la vapeur d'eau de préférence supérieure à 500 g/m²/24h, notamment comportant du polyuréthane, cette couche de protection étant de préférence translucide, de préférence la couche de protection (60) étant assemblée à l'article au moyen d'un adhésif, de préférence discontinu.

15. Article selon l'une quelconque des revendications précédentes, étant un pansement, notamment conditionné à l'état stérile.

## Patentansprüche

1. Gegenstand (1) für die Anwendung auf der Haut, den Schleimhäuten oder einer Wunde, um dort eine Flüssigkeit, insbesondere ein Exsudat, zu absorbieren, der Folgendes umfasst:
- eine distale Absorptionsschicht (20),
- eine Mehrzahl getrennter visueller Indikatoren (50), die im Fall eines Kontakts mit der Flüssigkeit das Aussehen verändern, wobei diese visuellen Indikatoren (50) in Kontakt mit der distalen Absorptionsschicht (20) stehen,
- eine Grenzfläche (30), die dazu bestimmt ist, mit der Haut oder den Schleimhäuten in Kontakt zu treten,
- zwischen der Grenzfläche (30) und der distalen Absorptionsschicht (20) mindestens eine an der Grenzfläche (30) anliegende proximale Absorptionsschicht (10),
- zwischen der proximalen Absorptionsschicht (10) und der distalen Absorptionsschicht (20) mindestens eine Wasserbarriereschicht (40), die mit mindestens einer, vorzugsweise einzigen und/oder exzentrischen, Passage (41) versehen ist, wodurch Flüssigkeit, die die proximale Absorptionsschicht (10) überquert hat, die distale Absorptionsschicht (20) erreichen kann,
wobei das Wasserabsorptionsvermögen der proximalen Absorptionsschicht (10) größer ist als das der distalen Absorptionsschicht (20).

2. Gegenstand nach Anspruch 1, wobei die Passage(n) (41) jeweils eine größere Querausdehnung (t) zwischen 100 µm und 1 cm, besser zwischen 100 µm und 5 mm, aufweisen.

3. Gegenstand nach dem vorhergehenden Anspruch, wobei die visuellen Indikatoren (50) in einem konstanten Abstand in mindestens einer Richtung, insbesondere in einem Abstand (*W*) zwischen 5 mm und 2 cm, angeordnet sind.

4. Gegenstand nach einem der vorhergehenden Ansprüche, wobei die visuellen Indikatoren (50) aus Pastillen bestehen, die auf der distalen Absorptionsschicht angeordnet sind.

5. Gegenstand nach einem der vorhergehenden Ansprüche, wobei die visuellen Indikatoren (50) voneinander durch ein hydrophobes Material getrennt sind.

6. Gegenstand nach einem der vorhergehenden Ansprüche, wobei die distale Absorptionsschicht (20) einen porösen hydrophilen Schaum, vorzugsweise einen Polyurethan-Schaum, enthält.

7. Gegenstand nach einem der vorhergehenden Ansprüche, wobei die die Barriereschicht (40) aus einem Film eines undurchlässigen Materials, insbesondere aus einem Film eines thermoplastischen Werkstoffs, insbesondere Polyurethan, besteht.

8. Gegenstand nach einem der vorhergehenden Ansprüche, wobei die proximale Absorptionsschicht (10) einen Schaum eines hydrophilen porösen Materials, insbesondere Polyurethan, umfasst.

9. Gegenstand nach einem der vorhergehenden Ansprüche, wobei das Wasserabsorptionsvermögen der proximalen Absorptionsschicht (10) zweimal so groß, besser fünfmal so groß, wie das der distalen Absorptionsschicht (20) ist.

10. Gegenstand nach einem der vorhergehenden Ansprüche, wobei die Grenzfläche (30) durch eine Kontaktschicht definiert ist, die vorzugsweise aus einem Vliesmaterial besteht, wobei der Gegenstand vorzugsweise zwischen der Kontaktschicht und der proximalen Absorptionsschicht (10) mindestens eine erste Verteilschicht (71) umfasst.

11. Gegenstand nach dem vorhergehenden Anspruch, der zwischen der proximalen Absorptionsschicht (10) und der Barriereschicht (40) eine zweite Verteilschicht (72) umfasst, wobei die zweite Verteilschicht (72) vorzugsweise in einer Zone (74), die sich gegenüber dem Umfang der Passage oder der Passagen (41) befindet und die gegebenenfalls eine Durchbrechung (73) gegenüber der Passage oder den Passagen (41) aufweist, lokal hydrophob gemacht worden ist, insbesondere durch eine PVC-Beschichtung.

12. Gegenstand nach einem der Ansprüche 10 und 11, wobei die Verteilschicht oder -schichten (71, 72) ein hydrophiles Textil oder Vliesmaterial, insbesondere auf der Basis von Polyesterfasern oder Polyolefinfasern, umfasst/umfassen.

13. Gegenstand nach einem der vorhergehenden Ansprüche, wobei die Barriereschicht (40) eine Wasserdampfdurchlässigkeit von mehr als oder gleich 500 g/m²/24 Std. aufweist.

14. Gegenstand nach einem der vorhergehenden Ansprüche, der eine Schutzschicht (60) umfasst, die die distale Absorptionsschicht (20) überlagert, wasserundurchlässig ist und eine Wasserdampfdurchlässigkeit von vorzugsweise mehr als 500 g/m²/24 Std. aufweist, die insbesondere Polyurethan umfasst, wobei diese Schutzschicht vorzugsweise durchsichtig ist, wobei die Schutzschicht (60) vorzugsweise mithilfe eines Klebstoffs, vorzugsweise diskontinuierlich, mit dem Gegenstand verbunden ist.

15. Gegenstand nach einem der vorhergehenden Ansprüche, bei dem es sich um einen, insbesondere sterilen, Wundverband handelt.

## Claims

1. An article (1) to be applied to the skin, the mucous membranes or a wound, in order to absorb therein a liquid, especially an exudate, comprising:
- a distal absorbent layer (20),
- a plurality of discrete visual indicators (50) that change appearance in the event of contact with the liquid, this or these visual indicator(s) (50) being in contact with the distal absorbent layer (20),
- an interface (30) intended to contact the skin or the mucous membranes,
- between the interface (30) and the distal absorbent layer (20), at least one proximal absorbent layer (10) adjacent to the interface (30),
- between the proximal absorbent layer (10) and the distal absorbent layer (20), at least one layer (40) forming a barrier to water provided with at least one passage (41), preferably being unique an/or off-center, enabling the liquid that has penetrated the proximal absorbent layer (10) to reach the distal absorbent layer (20),
the water absorption capacity of the proximal absorbent layer (10) being superior to the water absorption capacity of the distal absorbent layer (20).

2. The article as claimed in claim 1, the passage(s) (41) each having a larger transverse dimension (*t*) between 100 µm and 1 cm, better still between 100 µm and 5 mm.

3. The article as claimed in the preceding claim, the visual indicators (50) being positioned with a constant spacing in at least one direction, especially with a spacing (*W*) between 5 mm and 2 cm.

4. The article as claimed in any one of the preceding claims, the visual indicator(s) (50) consisting of pellets deposited on the distal absorbent layer.

5. The article as claimed in any one of the preceding claims, the visual indicators (50) being separated from one another by a hydrophobic material.

6. The article as claimed in any one of the preceding claims, the distal absorbent layer (20) comprising a hydrophilic porous foam, preferably a polyurethane foam.

7. The article as claimed in any one of the preceding claims, the barrier-forming layer (40) consisting of a film of an impermeable material, especially a film of a thermoplastic material, in particular of polyurethane.

8. The article as claimed in any one of the preceding claims, the proximal absorbent layer (10) comprising a foam of a hydrophilic porous material, especially of polyurethane.

9. The article as claimed in any one of the preceding claims, the water absorption capacity of the proximal absorbent layer (10) being greater than that of the distal absorbent layer (20), especially at least 2 times greater, better still at least 5 times greater.

10. The article as claimed in any one of the preceding claims, the interface (30) being defined by a contact layer, preferably consisting of a nonwoven, the article comprising preferably between the contact layer and the proximal absorbent layer (10), at least one first distribution layer (71).

11. The article as claimed in the preceding claim, comprising, between the proximal absorbent layer (10) and the barrier-forming layer (40), a second distribution layer (72), the second distribution layer (72) being preferably rendered hydrophobic locally, especially by coating with PVC, over an area (74) located facing the periphery of the passage(s) (41) and optionally having a hole (73) facing the passage(s) (41).

12. The article as claimed in any one of claims 10 and 11, the distribution layer(s) (71,72) comprising a hydrophilic textile or nonwoven, especially based on polyester fibers or polyolefin fibers.

13. The article as claimed in any one of the preceding claims, the barrier-forming layer (40) having a water vapor permeability greater than or equal to 500 g/m²/24 h.

14. The article as claimed in any one of the preceding claims, comprising a protective layer (60), that is superimposed on the distal absorbent layer (20), is impermeable to water and that has a water vapor permeability preferably greater than 500 g/m²/24 h, especially comprising polyurethane, this protective layer preferably being translucent, the protective layer (60) being preferably joined to the article by means of an adhesive, preferably a discontinuous adhesive.

15. The article as claimed in any one of the preceding claims, being a dressing, especially a sterilely packaged dressing.
